# EUROPEAN PATENT APPLICATION

(11) **EP 4 389 113 A1**
(43) Date of publication of application: **26.06.2024**
(21) Application number: 22306996.4
(22) Date of filing: 22.12.2022
(51) Int. Cl.: A61K 9/00, A61K 9/16, A61K 9/10

(54) **DOSAGE FORM FOR INTRAMEDULLARY INJECTION OR IMPLANTATION COMPRISING ELTROMBOPAG FOR USE IN THE IMPROVEMENT OF HEMATOPOIETIC STEM CELL TRANSPLANTATION**

(71) Applicant: PK MED, 75013 Paris (FR)
(72) Inventor: The designation of the inventor has not yet been filed
(74) Representative: Cabinet Nony

(57) **Abstract**

The present invention relates to a pharmaceutical composition comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a salt thereof, wherein it is suitable for intramedullary injection or implantation. The present invention also relates to a kit comprising, in separate compartments (i) an aqueous injection vehicle and (ii) a controlled release dosage form, an immediate release dosage form or a mixture thereof comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a salt thereof, wherein the pharmaceutical composition optionally comprises an excipient, for preparing a pharmaceutical composition suitable for an intramedullary injection. This composition is useful for intramedullary injection or implantation in a patient prior to or during or after an autologous or allogeneic HSCT, for improving the homing, engraftment and long - term expansion and proliferation of hematopoietic stem cells.

## Description

The present invention relates to the field of the treatment of non-malignant blood disorders such as hemoglobinopathies, or malignant hemopathies by autologous or allogeneic hematopoietic stem cell transplantation (HSCT) by Eltrombopag or a derivative or a conjugate or a pharmaceutically acceptable salt thereof. More particularly, the present invention concerns a pharmaceutical composition suitable for intramedullary injection or intramedullary implantation comprising Eltrombopag (EPAG) or a derivative or a conjugate or a pharmaceutically acceptable salt thereof. The present invention also concerns this pharmaceutical composition for its therapeutic uses, in particular for improving the homing of hematopoietic stem cells (HSCs) after an autologous or allogeneic HSCT and improving the treatment of malignant hemopathies and non-malignant blood disorders, such as hemoglobinopathies, primary immunodeficiencies, autoimmune diseases or inborn errors of metabolism.

### BACKGROUND

Hematopoietic stem cell transplantation, also known as bone marrow transplant, involves the administration of healthy hematopoietic stem cells from a donor to patients with depleted or dysfunctional bone marrow. The HSC donor can be either the patient itself (autologous HSCT) or a selected donor (allogenic HSCT). This procedure increases bone marrow function and leads to either destruction of malignant tumor cells or to generation of functional cells that can replace the dysfunctional ones. HSCT requires some degree of bone marrow (BM) ablation of the patient, known as conditioning, a preparative regimen administered to the patients undergoing HSCT before the infusion of the donor cells in order to induce immunosuppression of the patient (to ensure engraftment and limit rejection and GvHD), eradicate any hematological malignancy and to create space in the BM niche for the new donor cells to engraft.

Allogeneic HSCT uses donor cells obtained from healthy donors whose human leukocyte antigens (HLA) are acceptable matches to the patient's. The stem cell donor may be related to the patient, as in HLA-matched sibling donors, which remain to date the preferred donors, or as in haploidentical donors sharing half of the HLA with the patient, or they may be an unrelated volunteer. Allogenic HSCT has usually been restricted to younger patients in good general condition because of the increased risk of regimen-related toxicity and graft versus host disease (GVHD) with advanced age. In younger patients, the likelihood of having a matched sibling donor available is also increased.

Hematologic malignancies are generally diseases of the elderly, with a median of age for the patients of around 65-70 years. Acute myeloid leukemia (AML) is a genetically heterogeneous disorder characterized by the accumulation of somatically acquired genetic changes in hematopoietic progenitor cells that alter normal mechanisms of self-renewal, proliferation, and differentiation. Allogeneic HSCT is frequently performed for the second phase of AML treatment, called post-remission or consolidation phase, and has been found to reduce the risk of leukemia coming back more than standard consolidation chemotherapy. Importantly, elderly patients may not be able to tolerate high-dose standard consolidation chemotherapy, thus HSCT using reduced conditioning may be preferred. However, allogenic HSCT is more likely to have serious complications, including an increased risk of death. In addition, elderly patients do not usually have an available HLA-matched sibling donor and the use of haploidentical donors for transplantation is required, which increases the risk of GVHD after transplantation. Both the use of reduced conditioning and haplo donors decrease the rate of successful HSCT, therefore, a therapy increasing the efficacy rate of HSCT could allow elderly patients to successfully undergo consolidation treatment and to benefit from a reduced risk of leukemia relapse. In addition, allogenic HSCT is also the only curative therapy for other malignant disorders such as advanced myelodysplastic syndromes (MDS).

Hemoglobinopathies are a group of inherited disorders caused by alteration of the genes encoding for hemoglobin (Hb) and hence primarily affecting red blood cells. Among this group, sickle cell disease (SCD) and β-thalassemia major (βThal) are the most prevalent diseases and are caused respectively by abnormalities in the Hb structure or in the production of Hb. Allogeneic HSCT remains the only established curative treatment for SCD and β-Thal but major barriers still prohibit its use, particularly in adults. The highest efficacy and safety of this process is only achieved when HLA-matched sibling donors are available, which is only the case for less than 20% of patients, and transplantation is performed at a young age. The use of haploidentical donors, available in more than 80% of patients can overpass this limitation in donor availability, but entails a lower efficacy and safety, which is why haplo donors are still not used outside the context of clinical trials. Additionally, conditioning regimen-related toxicity especially limits the access to HSCT of adult patients frequently already presenting co-morbidities caused by the development of the disease. However, if the intensity of the conditioning is reduced, the efficacy of the transplantation is decreased.

Therefore, if an increase in the efficacy rate of allogenic HSCT for both malignant and non-malignant indications could be achieved, this would allow the safer use of haplo donors and a reduced intensity conditioning, especially benefiting adult patients.

In autologous HSCT the bone marrow products are collected from the patient and are reinfused after purification methods. The advantages include no GVHD and better engraftment. However, bone marrow products may contain abnormal cells that can cause relapse in the case of malignancy. Therefore, allogenic transplantation is preferred for many malignant indications such as leukemias and MDS since donor infused cells are free of contaminating tumor cells and therefore there is generally a lower risk for disease recurrence. Autologous transplants have been used more often in solid tumors, lymphoma, and myeloma, although allogenic transplantations combined with reduced conditionings are under evaluation for these indications, as a means to induce graft-versus-malignancy effect. Autologous HSCT is also used in non-malignant indications such as autoimmune diseases and in hemoglobinopathies when gene therapy is performed. One of the main limitations of gene therapy for the treatment of hemoglobinopathies is the decreased engraftment of the *ex vivo* manipulated cells, for which an increased rate of engraftment after transplantation could be of great benefit.

The therapeutical success of HSCT critically depends on the homing and engraftment of a sufficient number of the donor hematopoietic stem cells (HSCs) to the patient's bone marrow (BM) followed by expansion and differentiation of the cells allowing to reconstitute and sustain hematopoiesis in the patient. However, preclinical studies suggest that only between 1% and 10% of the intravenously infused HSCs find their way to the patient's BM, while the majority of the cells are tethered by other organs (van Hennik et al., Blood. 1999;94(9):3055-61) Therefore, it remains a need to increase the fraction of transplanted HSCs that effectively home and engraft in the patient's BM to improve the success rate of HSCT.

It is commonly accepted that SDF-1/CXCR4 signaling axis is one of the primary mediators of hematopoietic stem and progenitor cell homing to the BM following transplantation. SDF-1, also known as CXCL12, is a chemokine constitutively expressed by BM stromal cells and osteoblasts, while its receptor CXCR4 is expressed by HSCs. Many studies have shown that inhibitors for the SDF-1/CXCR4 axis inhibit transplanted HSC homing to the BM and induce HSC mobilization from the bone marrow to the circulation (Dar et al., Exp Hematol. 2006;34(8):967-75). In vitro, HSCs have been shown to migrate towards increasing gradients of SDF-1 (Andreas et al., Trends Biotechnol. 2014;32(9):483-92). In addition, SDF-1 has been shown to also play an important role in the maintenance and retention of HSCs within the BM (Greenbaum et al., Nature. 2013;495(7440):227-30). More interestingly, recent studies have shown an impaired production of BMSC-derived SDF-1 in preclinical models for hematological diseases, causing decreased SDF-1 levels in the BM extracellular fluid, which was accompanied by a decreased HSC maintenance in the BM and increased HSC mobilization, as well as decreased HSC engraftment in the BM after HSC transplantation (Tang et al., Blood. 2021;138(24):2570-2582; Hanoun et al., Cell Stem Cell. 2014;15(3):365-375). Moreover, the conditioning regimen or chemotherapy that patients need to undergo before HSCT affect the state of the BM niche, producing, among other effects, a local increase in proteases, able to reduce the half-life of chemokines such as SDF-1 (Zhang et al., Sci Rep. 2016;6:37827). Therefore, there remains a need to protect the SDF-1 locally in the BM and to restore the HSC niche homeostasis to enhance homing and engraftment. Subsequent survival of HSC progenitors that have homed to the BM, followed by their self-renewal and differentiation, is also a critical point for long-term success of the HSCT therapy.

Eltrombopag olamine (EPAG olamine) is a small molecular human thrombopoietin receptor (TPO-R) agonist, with the formula 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine). It is a drug that presents multiple mechanisms of action. The first identified effect corresponds to the stimulation of megakaryocyte (MK) progenitor expansion and differentiation through binding to the TPO-R. EPAG binding to the TPO-R also promotes HSC survival and is not inhibited in an inflammatory environment, in contrast to thrombopoietin effect. EPAG has also shown interesting iron chelating properties inducing a stimulatory effect on stem cell renewal and an anti-proliferative effect on leukemic cell lines.

EPAG is currently indicated for the treatment of immune thrombocytopenia (ITP), hepatitis C-associated thrombocytopenia and severe aplastic anemia (SAA). In addition, EPAG has also achieved positive results in the treatment of myelodysplastic syndrome and poor graft function post HSCT.

EPAG is currently commercially available as PROMACTA^{®} oral tablets or powder for suspension at doses ranging from 12,5 mg to 75 mg. Its use is associated with clinically significant adverse reactions such as hepatotoxicity and thrombotic/thromboembolic complications.

### Prior art

WO2016/201354 discloses a method of treating cancer comprising the administration of an antifungal agent, a TPO receptor agonist or a combination thereof and acting through inhibition of dioxygenase. However, examples are limited to immediate-release injectable formulations and immediate-release oral tablets. Additionally, it clearly comes out that the proposed method does not pertain to a scheme of treatment comprising HSC transplantation.

WO2007/145227 relates to the administration of an agonist agent for the TPO receptor after HSCT to enhance the engraftment, growth and differentiation of transplanted cells in the bone marrow. The agent is preferentially administered parenterally by injection but nothing is said about intramedullary injection of a dosage form prior to, during or after transplantation.

Use of a thrombopoietin receptor agonist, including EPAG, to promote the homing of HSCs after bone marrow transplantation has been reported in CN105412930A. A large set of administration routes is mentioned, however the unique example is limited to administration of an immediate-release EPAG solution by oral gavage in mice.

Therefore, it remains a need to develop novel dosage forms and route of administrations for EPAG in order to improve control over EPAG local concentration in the bone marrow and thus increase the fraction of transplanted HSCs that will home to the bone marrow.

The inventor surprisingly found that injection or implantation of a dosage form of EPAG or a derivative or a conjugate or a pharmaceutically acceptable salt thereof, in particular EPAG olamine, directly in the bone marrow prior to, during or after receiving HSCT enhances homing and engraftment of the transplanted cells and thus increases transplantation success. More particularly, the inventor surprisingly found that the use of EPAG or a derivative or a conjugate or a pharmaceutically acceptable salt thereof, in particular EPAG olamine, in a composition suitable for intramedullary injection or intramedullary implantation prior to, during or after receiving HSCT inhibit protease activity and stabilizes SDF-1 local concentration, which improves homing and engraftment of the transplanted cells and thus increases transplantation success.

### SUMMARY OF THE INVENTION

According to a first aspect, herein is provided a pharmaceutical composition comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, wherein it is suitable for intramedullary injection or intramedullary implantation.

According to a second aspect, herein is provided a pharmaceutical composition under the form of a powder comprising controlled release microparticles comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, and a controlled release polymeric matrix, wherein
- said controlled release microparticles have a mean particle size greater than or equal to 2 µm, in particular ranging from 2 µm to 150 µm, more particularly ranging from 10 µm to 100 µm, even more particularly ranging from 10 µm to 80 µm,
- said controlled release polymeric matrix comprises at least a poly(lactic-co-glycolic acid) copolymer, and
- the drug loading content or percentage weight ratio between the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof and the total weight of the microparticles ranges from 2% to 45%, in particular from 5% to 40%, more particularly from 10% to 35%.

According to a third aspect, herein is provided a kit or article of manufacture comprising, in separate compartments (i) an aqueous injection vehicle and (ii) a controlled release dosage form, an immediate release dosage form or a mixture of a controlled release dosage form and an immediate release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, in particular as defined in the present disclosure or powder as defined in the present disclosure, wherein the pharmaceutical composition optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intramedullary injection.

According to a fourth aspect, herein is provided a pharmaceutical composition as defined in the present disclosure or as obtained by mixing the two compartments of the kit as defined in the present disclosure, for use once or several times by intramedullary injection or intramedullary implantation in a patient prior to or during or after an autologous or allogeneic hematopoietic stem cell transplantation (HSCT), in particular a human patient, for improving the homing, engraftment and long - term expansion and proliferation of hematopoietic stem cells.

As mentioned above, the pharmaceutical composition as defined in the present disclosure suitable for intramedullary injection or intramedullary implantation in a patient prior to or during or after an autologous or allogeneic hematopoietic stem cell transplantation (HSCT), is effective in improving the homing, engraftment and long - term expansion and proliferation of hematopoietic stem cells prior to allogeneic HSCT, in particular for decreasing graft failure, for decreasing the occurrence of Poor Graft function (PGF), for decreasing the occurrence of Graft-versus-host disease (GvHD), for enhancing overall survival and donor chimerism, and/or for promoting hematopoietic recovery and hematopoietic stem cell transplantation success rate, and also in preventing and/or treating non-malignant blood disorders such as severe aplastic anemia, and hemoglobinopathies, in particular Sickle cell disease and beta-Thalassemia and/or in the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia and/or in the prevention and/or treatment of primary immunodeficiencies, autoimmune diseases or inborn errors of metabolism.

### BRIEF DESCRIPTION OF THE FIGURES

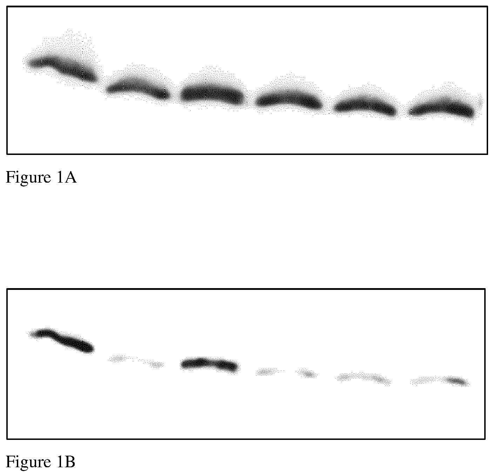

Figures 1A and 1B represent a western blot analysis showing the inhibitory effect of Eltrombopag olamine on the proteolysis of SDF-1 by MMP-9 (Matrix Metalloproteinase-9). Western blot images of total SDF-1 (top panel or panel A) and N-terminally intact SDF-1 (bottom panel or panel B). From left to right in each panel A and panel B, Lanes are named respectively Lane1 to Lane 6. Lane 1 corresponds to SDF-1 alone, Lane 2 corresponds to SDF-1 plus MMP-9, Lane 3-6 correspond to SDF-1 plus MMP-9 plus Eltrombopag olamine at decreasing eltrombopag olamine concentrations (50 µM, 10 µM, 1 µM and 0.1 µM).

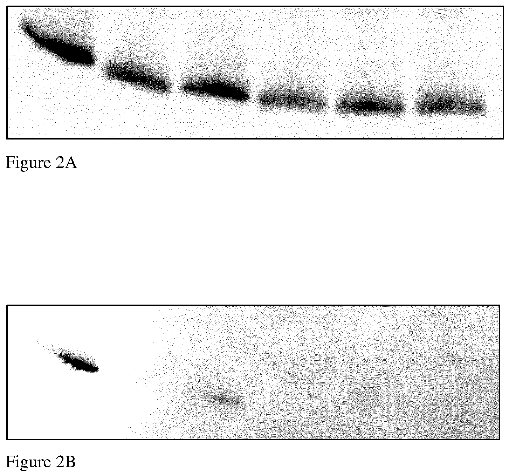

Figures 2A and 2B represent a western blot analysis showing the inhibitory effect of eltrombopag olamine on the proteolysis of SDF-1 by MMP-8 (Matrix Metalloproteinase-8). Western blot images of total SDF-1 (top panel or panel A) and N-terminally intact SDF-1 (bottom panel or panel B). From left to right in each panel A and panel B, Lanes are named respectively Lane1 to Lane 6. Lane 1 corresponds to SDF-1 alone, Lane 2 corresponds to SDF-1 plus MMP-8, Lane 3-6 correspond to SDF-1 plus MMP-8 plus Eltrombopag olamine at decreasing eltrombopag olamine concentrations (50 µM, 10 µM, 1 µM and 0.1 µM).

### DETAILED DESCRIPTION OF THE INVENTION

As apparent from the BACKGROUND paragraph above, the inventors have found that the intramedullary injection or intramedullary implantation of a pharmaceutical composition comprising EPAG or a derivative or a conjugate or a pharmaceutically acceptable salt thereof such as EPAG olamine prior to or during or after an autologous or allogeneic HSCT allows to improve the homing, engraftment and long - term expansion and proliferation of HSCs.

More particularly, the inventors have found that the intramedullary injection of a pharmaceutical composition under the form of a suspension and that the intramedullary implantation of a pharmaceutical composition under the form of an implant for a release (immediate release, controlled release or mixture of immediate and controlled release) of EPAG or a derivative or a conjugate or a pharmaceutically acceptable salt thereof such as EPAG olamine prior to or during or after HSCT increases the fraction of transplanted HSCs that effectively home and engraft within the receiver's bone marrow resulting in a faster return to normal hematopoiesis and overall higher transplantation success.

In particular, the inventors have found that the intramedullary injection or the intramedullary implantation of EPAG or a derivative or a conjugate or a pharmaceutically acceptable salt thereof such as EPAG olamine allows to improve the local concentration of EPAG or a derivative or a conjugate or a pharmaceutically acceptable salt thereof such as EPAG olamine in such a manner that its local concentration is higher than 14 µM, which is the maximum plasmatic concentration after oral administration of a 50 mg tablet.

As mentioned above, and as apparent in the illustrative part herein after (examples 4 and 5), the inventors have demonstrated that EPAG or a derivative or a conjugate or a pharmaceutically acceptable salt thereof allows to inhibit protease activity and stabilizes SDF-1 local concentration. This is the first time such a mechanism of action is demonstrated in connection to EPAG or a derivative or a conjugate or a pharmaceutically acceptable salt thereof.

### Definitions

As used herein, the term "patient" refers to a human or a human child, which is afflicted with, or has the potential to be afflicted with one or more diseases and conditions described herein.

The identification of those patients who are in need of treatment of herein-described diseases and conditions is well within the ability and knowledge of one skilled in the art. A physician skilled in the art can readily identify, by the use of clinical tests, physical examination, medical/family history or biological and diagnostic tests, those patients who are in need of such treatment.

In the context of the invention, the term "treating" or "treatment", as used herein, means reversing, alleviating, decreasing, inhibiting the progress of non-malignant blood disorders such as severe aplastic anemia, and hemoglobinopathies, in particular Sickle cell disease and beta-Thalassemia, and/or of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia, and/or of primary immunodeficiencies, autoimmune diseases or inborn errors of metabolism.

In the context of the invention, the term "preventing" or "prevention", as used herein, means reducing the likelihood of occurrence of, or delaying onset of non-malignant blood disorders such as severe aplastic anemia, and hemoglobinopathies, in particular Sickle cell disease and beta-Thalassemia, and/or of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia, and/or of primary immunodeficiencies, autoimmune diseases or inborn errors of metabolism.

In the context of the invention, the expression "improving the homing, engraftment and long-term expansion and proliferation of hematopoietic stem cells (HSC)" means fostering the ability of exogenously administered HSCs to locate, seed and engraft within the receiver's bone marrow thus increasing the total number of transplanted HSCs capable of self-renewal and multipotential differentiation and ultimately participating in the reconstitution of a viable hematopoietic system.

As used herein, an "effective amount" refers to an amount of a compound of the present invention which is effective in reducing, eliminating, treating or controlling the symptoms of the herein-described diseases and conditions, *i.e.* non-malignant blood disorders such as severe aplastic anemia, and hemoglobinopathies, in particular Sickle cell disease and beta-Thalassemia, or malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia, and/or of primary immunodeficiencies, autoimmune diseases or inborn errors of metabolism.. The term "controlling" is intended to refer to all processes wherein there may be a slowing, interrupting, arresting, or stopping of the progression of the diseases and conditions described herein, but does not necessarily indicate a total elimination of all diseases and condition symptoms.

The term "treatment-effective amount" refers to a concentration of compound that is effective in treating non-malignant blood disorders such as severe aplastic anemia, and hemoglobinopathies, in particular Sickle cell disease and beta-Thalassemia, and/or malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia, and/or of primary immunodeficiencies, autoimmune diseases or inborn errors of metabolism.

In the sense of the present invention, the expression "local concentration of a product", in particular "local concentration of EPAG", more particularly in equivalent EPAG free acid means the concentration of the product, that is to say the concentration of EPAG at the site of injection or at the site of implantation in the human body, in particular in the bone marrow of the patient.

As used herein, the term "pharmaceutically acceptable" refers to those compounds, materials, excipients, compositions or dosage forms which are, within the scope of sound medical judgment, suitable for contact with the tissues of human beings without excessive toxicity, irritation, allergic response or other problem complications commensurate with a reasonable benefit/risk ratio.

The term "pharmaceutically acceptable carrier, adjuvant, or vehicle" may refer to any pharmaceutically acceptable excipient, such as a non-toxic carrier, adjuvant, or vehicle that does not destroy the pharmacological activity of the compound with which it is formulated.

The terms "immediate release composition" or "immediate release dosage form" mean that the composition or the dosage form allows an immediate release into the body of a specified amount of an active ingredient.

The terms "controlled release composition" or "controlled release dosage form" or "controlled release microparticle" or "controlled release polymeric matrix" mean that the composition or the dosage form or the microparticle or the polymeric matrix allows a release into the body of a specified amount over a specified period of time of an active ingredient, namely a specific pharmacokinetic profile. The term "controlled release" encompasses all the type of releases that are modified in comparison to an immediate release. In other words, the term "controlled release" is equivalent to "modified release" and encloses extended releases as defined herein after as well as delayed release and pulse releases.

The terms "extended release", "prolonged release" and "sustained release" are considered equivalent in the framework of the present invention. This type of release means that the release is prolonged over time in comparison to an immediate release, i.e. it means that the active ingredient is released slowly over time, allowing a less frequent intake of the drug for the patient. In other words, the active ingredient is progressively released over a specific period of time, generally aiming at less side effects as well by decreasing the maximal concentration.

The term "drug loading content" means the mass ratio (also named weight ratio) of the drug in the microparticles to the mass of microparticles.

The term "mean particle size" or D50 means the particle diameter in microns that splits the particle volume distribution with half above and half below this diameter.

The term "D10=x µm" as mainly used in the example means that 10% of the particles have a size of x µm or less.

The term "D90=y µm" as mainly used in the example means that 90% of the particles have a size of y µm or less.

A controlled or modified release may of course result from the combination of an immediate release and a controlled release.

### ELTROMBOPAG AND PHARMACEUTICALLY ACCEPTABLE SALTS THEREOF

As mentioned above, the pharmaceutical composition according to the present disclosure comprises 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof.

3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid is known as being useful as an agonist of the TPO (thrombopoietin) receptor that interacts with the transmembrane domain of the human TPO-receptor and is known by the generic name eltrombopag (C₂₅H₂₂N₄O₄, CAS: [496775-61-2]). Eltrombopag has a molecular weight of 442.5 g/mol and can be represented by the following formula:

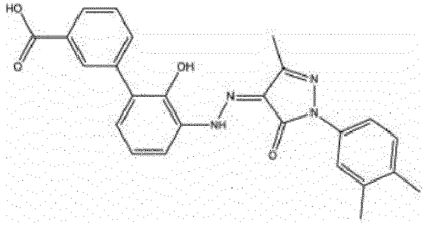

Eltrombopag may exist in different crystal forms for the free acid, including a large number of hydrates and solvates, as well as different cationic salt forms, all said forms being encompassed within the scope of the invention.

According to a preferred embodiment, the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a pharmaceutically acceptable salt thereof is under the form of bis-monoethanolamine salt of 3'-[(2Z)-[1-(3,4-dimethylphenyl)-1,5-dihydro-3-methyl-5-oxo-4H-pyrazol-4-ylidene]hydrazino]-2'-hydroxy-[1,1 '-biphenyl]-3-carboxylic acid.

The bis-monoethanolamine salt of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid (also known as bis-monoethanolamine salt of eltrombopag or as 3'-{(2Z)-2-[1-(3,4-dimethylphenyl)-3-methyl-5-oxo-1,5-dihydro-4H-pyrazol-4-ylidene]hydrazino}-2'-hydroxy-3-biphenylcarboxylic acid - 2-aminoethanol (1:2)) is generically known as eltrombopag olamine. Eltrombopag olamine (C₂₅H₂₂N₄O₄ • 2(C₂H₇NO), CAS: [496775-62-3]) has a molecular weight of 564.65 g/mol and can be represented by the following formula:

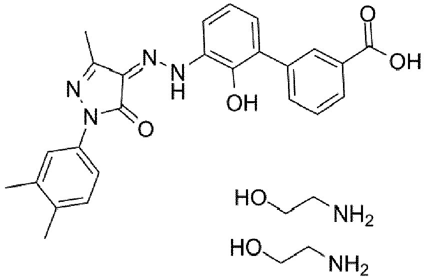

Eltrombopag olamine is marketed under the trade name Promacta^{®} in the United States and Revolade^{®} outside the United States.

Derivatives of Eltrombopag comprise any derivatives such as esters for example methyl, ethyl, pivaloyloxymethyl, and the like for -COOH, and acetate maleate and the like for -OH, and any esters well known in the art by the skilled person.

Derivatives of Eltrombopag can also comprise protected forms of Eltrombopag in which one or more functional groups such as the OH function(s) is(are) protected. The protection of hydroxy group or carboxylic group can be carried out by any groups and methods known and well described in the art for example, in "Protective Groups in Organic Synthesis" by Theodora W. Greene, Wiley-Interscience 1981, New York.

Conjugates of Eltrombopag comprise any conjugates such as an antibody-Eltrombopag conjugate, i.e. a polypeptide such as an antibody to which is covalently attached *via* a linker at least one molecule of Eltrombopag.

3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof suitable for the present invention can be comprised in a controlled release dosage form, an immediate release dosage form or a mixture of a controlled release dosage form and an immediate release dosage form.

In the pharmaceutical composition according to the present disclosure, 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof can be present in an amount ranging from 2 mg to 63 mg equivalent free acid per ml of the controlled release dosage form, the immediate release dosage form or the mixture of a controlled release dosage form and an immediate release dosage form, in particular from 8 mg to 55 mg equivalent free acid per ml, more particularly from 12 mg to 47 mg equivalent free acid per ml.

In the pharmaceutical composition according to the present disclosure, EPAG olamine can be present in an amount ranging from 2 mg to 80 mg per ml of the controlled release dosage form, the immediate release dosage form or the mixture of a controlled release dosage form and an immediate release dosage form, in particular from 10 mg to 70 mg per ml, more particularly from 15 mg to 60 mg per ml.

### CONTROLLED-RELEASE DOSAGE FORM

As mentioned above, 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof can be comprised in a controlled release dosage form.

According to one embodiment, the controlled release dosage form comprising Eltrombopag or a derivative or a conjugate or a pharmaceutically acceptable salt thereof is under the form of an in-situ forming depot, a hydrogel, a microporous implant, a solid implant or microparticles, in particular microparticles comprising a controlled-release polymeric matrix, or multivesicular liposomes.

In one embodiment, the controlled release dosage form is under the form of an in-situ forming depot or implant. An in-situ forming depot or implant, such as pH-induced, thermally-induced or solvent exchange-induced gelling systems comprising eltrombopag or a conjugate or a derivative or a salt thereof may be prepared according to techniques known to the man skilled in the art, as in particular described in Ibrahim TM et al. An overview of PLGA in-situ forming implants based on solvent exchange technique: effect of formulation components and characterization. Pharm Dev Technol. 2021 Sep;26(7):709-728 and in S. Kempe et al. In-situ forming implants-an attractive formulation principle for parenteral depot formulations Journal of Controlled Release 2012, 161:668.

In another embodiment, the controlled release dosage form is under the form of a hydrogel. A hydrogel may be prepared according to techniques known by the man skilled in the art. In particular a hydrogel may be formed by dispersing the eltrombopag or a conjugate or a derivative or a salt thereof, optionally previously solubilized in water, under stirring with a hydrogel. Among suitable hydrogels, the following may be cited: sodium hyaluronate. When the pharmaceutical composition is under the form of a powder, water shall be removed by techniques known to the man skilled in the art, for example by dehydration or lyophilization. When needed they may be hydrated back reversibly.

In another embodiment, the controlled release dosage form is under the form of a microporous implant.

In another embodiment, the controlled release dosage form is under the form of solid implants, such as preformed porous or non-porous solid implants that can be administered via large needles, cannulas or microsurgery.

In one embodiment, the controlled release dosage form is under the form of microparticles. Said embodiments are described in detail herein after.

### CONTROLLED RELEASE MICROPARTICLES COMPRISING ELTROMBOPAG OR DERIVATIVES OR CONJUGATES OR SALTS THEREOF

The controlled release microparticles comprising eltrombopag or derivatives or conjugates or salts thereof may take the form of various microparticles, such as (i) microparticles comprising a polymeric matrix (also named controlled release polymeric matrix) or (ii) multivesicular liposomes.

According to one embodiment, the microparticles have a mean particle size equal or greater than 2 µm.

According to another embodiment, the microparticles have a mean particle size equal to or greater than 2 µm, in particular a mean particle size lower than 150 µm, more particularly lower than 100 µm, for example ranging from 10 µm to 100 µm, and even more particularly lower than 80 µm, for example between 2 and 80 µm or between 10 and 80 µm.

According to a preferred embodiment, the microparticles have a mean particle size equal to or greater than 2 µm, and in particular ranging from 2 µm to 150 µm, more particularly from 10 µm to 100 µm, even more particularly from 10 µm to 80 µm.

It is understood that these ranges refer to the mean size of all microparticles in a given population. The size of any given individual microparticle could be within a standard deviation above or below the mean size.

Within the context of the present invention, a "microparticle" means a particle of any shape and made of any material, in particular suitable for the intramedullary injection or the intramedullary implantation into the human body within a pharmaceutical composition, and in particular into the bone marrow, having a mean particle size greater than or equal to 2 µm, in particular ranging from 2 µm to 150 µm, more particularly from 10 µm to 100 µm, even more particularly from 10 µm to 80 µm.

The microparticles suitable for the pharmaceutical composition used according to the present invention may be selected form various types of microparticles such as microspheres, microparticle matrices, microsphere matrices, microcapsules, rods, wafers, pills fibers and pellets.

### (i) MICROPARTICLES COMPRISING A POLYMERIC MATRIX

The polymeric matrix may be chosen from various polymers suitable for obtaining controlled release microparticles as explained below. Such polymeric matrix is nontoxic to the human body.

According to one embodiment, the polymer forming the polymeric matrix is biodegradable and biocompatible.

Within the context of the present invention, a "biodegradable" material, refers to a material which degrades enzymatically or hydrolytically and for which there is proof that the degradation products are integrated into biomass and/or eliminated from the organism by metabolism or renal filtration.

Within the context of the present invention, a "biocompatible" material, refers to a material which is tolerated by the human body.

The nontoxic, biocompatible and biodegradable polymer may be natural or synthetic.

According to one embodiment, the controlled-release polymeric matrix according to the present invention comprises at least one biocompatible and biodegradable copolymer or polymer selected from a poly(lactic-co-glycolic acid) copolymer (also named PLGA or PLG), poly(caprolactone), poly(lactide) (also named PLA), poly(glycolide) (also named PGA), poly(lactide-co-caprolactone), poly(ethylene glycol), poly(ethylene oxide) (also named PEO), PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol), PVA-g-PLGA, poly (ether ester) multiblock copolymers, polyvinyl pyrrolidone, poly(methacrylate), PEO-PPO-PEO (pluronics, also named polyethylene oxide-polypropylene oxide-polyethylene oxide), gelatin, heparin, chondroitin sulfate; polysaccharides such as alginates, starch, chitosan, hyaluronic acid and dextrans, and any combinations thereof.

According to a particular embodiment, the controlled-release polymeric matrix comprises at least a poly(lactic-co-glycolic acid) copolymer.

According to said embodiment, the controlled-release polymeric matrix may comprise poly(lactic-co-glycolic acid) in an amount of greater than 70% by weight, with respect to the total weight of the polymeric matrix, in particular greater than 80% by weight, and even more particularly greater than 90% by weight.

Suitable polymers are not limited to those commercially available and known as RESOMER (Evonik Industries AG, Germany), LACTEL (Durect, USA), PURASORB (Corbion NV, The Netherlands), Viatel (Ashland, USA), EXPANSORB (Seqens, FRANCE).

Examples of suitable polymers are listed in Table A.

**Table A:**

| **Product name** | **Polymer** | **End group** | **Producer** |
|---|---|---|---|
| Resomer RG 502H | Poly(D,L-lactide-co-glycolide) 50:50 | acid | Evonik |
| Resomer RG 502 | Poly(D,L-lactide-co-glycolide) 50:50 | ester | Evonik |
| Resomer RG 503H | Poly(D,L-lactide-co-glycolide) 50:50 | acid | Evonik |
| Resomer RG 503 | Poly(D,L-lactide-co-glycolide) 50:50 | ester | Evonik |
| Lactel B6013-1 | Poly(D,L-lactide-co-glycolide) 50:50 | acid | Durect |
| Lactel B6017-1 | Poly(D,L-lactide-co-glycolide) 50:50 | ester | Durect |
| Lactel B6010-1 | Poly(D,L-lactide-co-glycolide) 50:50 | ester | Durect |
| Purasorb PDLG 5002A | Polv(D,L-lactide-co-glycolide) 50:50 | acid | Corbion |
| Purasorb PDLG 5002 | Poly(D,L-lactide-co-glycolide) 50:50 | ester | Corbion |
| Purasorb PDLG 5004A | Poly(D,L-lactide-co-glycolide) 50:50 | acid | Corbion |
| Purasorb PDLG 5004 | Poly(D,L-lactide-co-glycolide) 50:50 | ester | Corbion |
| Viatel | Poly(D,L-lactide-co-glycolide) 50:50 | acid/ester | Ashland |
| Expansorb DLG 2A | Poly(D,L-lactide-co-glycolide) 50:50 | acid | Seqens |
| Expansorb DLG 3A | Poly(D,L-lactide-co-glycolide) 50:50 | acid | Seqens |
| Expansorb DLG 5A | Poly(D,L-lactide-co-glycolide) 50:50 | acid | Seqens |
| Expansorb DLG 6A | Poly(D,L-lactide-co-glycolide) 50:50 | acid | Seqens |
| Expansorb DLG 2E | Poly(D,L-lactide-co-glycolide) 50:50 | ester | Seqens |
| Expansorb DLG 6E | Poly(D,L-lactide-co-glycolide) 50:50 | ester | Seqens |

The polymeric matrix may comprise PLGA and one or more additional polymer(s), copolymer(s) or mixture thereof. Said additional polymer(s), copolymer(s) or mixture thereof may be present in the polymer matrix in an amount ranging from 0 to 30% by weight, in particular from 0 to 20% by weight, and more particularly from 0 to 10% by weight, with respect to the total weight of the polymeric matrix.

Thus, according to another particular embodiment, the controlled-release polymeric matrix comprises at least a poly(lactic-co-glycolic acid) copolymer and at least one suitable additional biocompatible and biodegradable polymer or copolymer selected from a poly(lactide) distinct from poly(lactic-co-glycolic acid) copolymer, poly(caprolactone), poly(glycolide) distinct from poly(lactic-co-glycolic acid) copolymer, poly(lactide-co-caprolactone), poly(ethylene glycol), poly(ethylene oxide), PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol), PVA-g-PLGA, poly (ether ester) multiblock copolymers, polyvinyl pyrrolidone, poly(methacrylate), PEO-PPO-PEO (pluronics), gelatin, heparin, chondroitin sulfate; polysaccharides such as alginates, starch, chitosan, hyaluronic acid, and dextrans, and any combinations thereof,

According to another embodiment, the microparticles suitable for the present disclosure have a particle size distribution having a D50 value of not more than 150 µm, in particular of not more than 100 µm, and for example from 2 µm to 80 µm.

It is understood that these ranges refer to the mean size of all microparticles in a given population. The size of any given individual microparticle could be within a standard deviation above or below the mean size.

In one embodiment, the controlled release microparticles according to the present invention are PLGA microspheres. In other words, in said embodiment, the polymeric matrix does not comprise any additional polymer or copolymer.

When PLGA copolymers are implemented as a polymer matrix, they can have a wide range of molecular weights and monomer ratios of lactic to glycolic acid, in particular of 75:25 to 50:50, and even more particularly of 50:50. Any suitable method known in the art of making the polymer can be used and the molecular weights may typically range from 5 to 150 kDa, in particular from 5 to 80 kDa, and more particularly from 5 to 50 kDa, for example from 10 to 150 kDa, from 10 to 80 kDa or from 10 to 50 kDa.

According to a further particular embodiment, the polymer forming the polymeric matrix comprises PLGA, for example in an amount of 100% by weight, with respect to the total weight of the polymeric matrix, and the PLGA is defined by:
- A molecular weight between 5 and 80 kDa, and
- A lactide:glycolide molar ratio of 75:25 to 50:50.

According to a more particular embodiment, the polymer forming the polymeric matrix comprises PLGA, for example in an amount of 100% by weight, with respect to the total weight of the polymeric matrix, and the PLGA is defined by:
- A molecular weight between 5 and 50 kDa, and
- A lactide:glycolide molar ratio of 50:50.

According to one embodiment, PLGA is either carboxylic or ester endcapped, and is in particular carboxylic endcapped.

According to another embodiment, microparticle matrix can comprise, and even may consist in, a blend of two PLGA copolymers, in particular one of low molecular weight and one of high molecular weight.

The microparticle may further comprise pharmaceutically acceptable excipients for modulating the drug release profile such as medium chain triglycerides, poly(oxyethylene) sorbitan fatty acid esters (e.g. polysorbate 20, polysorbate 80), sorbitan fatty acid esters, cyclodextrins, lecithin, mannitol, sucrose, inorganic salts and mixtures thereof.

The polymeric matrix of the microparticles may comprise one or more excipient(s). Said excipient(s) may be present in the polymer matrix in an amount not exceeding 15% by weight, in particular not exceeding 10% by weight, and more particularly not exceeding 5% by weight, with respect to the total weight of the polymeric matrix.

According to one embodiment, the drug loading content or percentage weight ratio between the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or derivative or conjugate or a pharmaceutically acceptable salt thereof, in particular EPAG olamine, and the total weight of the microparticles ranges from 2% to 45%, in particular from 5% to 40%, more particularly from 10% to 35%.

### Manufacturing process for the obtention of the microparticles comprising a polymer matrix

Any process suitable to produce polymeric microparticles with a mean particle size ranging from 2 µm to 150 µm is considered appropriate in the framework of the present invention.

Among such manufacturing process emulsification-based processes, such as high-pressure homogenization, for example using rotor-stator homogenizer in a batch or continuous mode, or membrane emulsification, followed by organic solvent removal by extraction/evaporation may be cited.

As far as the general principal of such methods is concerned, an emulsion may be prepared and processed over a membrane with pores with a determined size. The obtained microspheres can then be collected after extraction and evaporation of the organic solvent, washed and freeze-dried.

According to one particular embodiment, the microparticles may be manufactured from an O/W direct emulsion or from a W/O/W double emulsion technique.

Schoubben, A., Ricci, M. & Giovagnoli, S. Meeting the unmet: from traditional to cutting-edge techniques for poly lactide and poly lactide-co-glycolide microparticle manufacturing. J. Pharm. Investig. 49, 381-404 (2019) reviews various methods to make PLGA microparticles that can be used in the framework of the present invention.

According to one particular embodiment, the microparticles may be manufactured from a Solid-in-Oil-in-Water (S/O/W) double emulsion technique.

Giovagnoli, S., et al.; Physicochemical characterization and release mechanism of a novel prednisone biodegradable microsphere formulation, J Pharm Sci. 97:303-317, (2008) describes an example of PLGA microparticles prepared *via* S/O/W emulsion technique. Other manufacturing approaches suitable for obtaining the microparticles according to the present invention are atomization by spinning disk, atomization by spray-drying, a fluidized bed coating, or combinations thereof.

Alternatively, the microparticles may be manufactured using drop-on-demand, drop-by-drop and jet break-up processes such as inkjet printing or microfluidics.

Alternatively, the microparticles may be manufactured using supercritical fluid technologies.

Alternatively, the microparticles may be manufactured using microfabrication methods, such as template and mold-based techniques, such as soft lithography.

All these manufacturing processes are well known by the man skilled in the art.

The hereabove cited manufacturing processes are well known to the man skilled in the art.

### (ii) MULTI VESICULAR LIPOSOMES

Multivesicular liposomes (MVL) are spherical particles with a mean diameter of 10-30 µm and composed of non-concentric multiple lipid bilayers arranged in a honeycomblike structure. These lipid layers enclose numerous water-filled aqueous compartments that can be used to encapsulate a water-soluble drug such as eltrombopag or derivative or conjugate or salts thereof.

MVLs are classically composed of at least one amphiphilic lipid and one neutral lipid. The amphiphilic lipid is selected from phospholipids for instance phosphatidylcholine or phosphatidylglycerol. The neutral lipid is selected from triglycerides having mono-unsaturated fatty acid ester moieties containing 14-18 carbons in the acyl chain (e.g. triolein, tripalmitolein), saturated fatty acid ester moieties containing 6 to 8 carbons in the acyl chain (e.g. tricaproin, tricaprylin) and mixtures thereof. Cholesterol can also be used in the composition.

MVLs are obtained by using a water-in-oil-in-water double emulsification process. In a first step, a water-in-oil emulsion is prepared by mixing phospholipids, triolein, tricaprylin and cholesterol solubilized in volatile and water-immiscible organic solvent with an aqueous solution containing the solubilized drug to be encapsulated. This first emulsion is then emulsified by mixing with a second aqueous solution to produce the water-in-oil-in-water emulsion. The energy needed to form the first and second emulsion can be provided either mechanically, by sonication or by combinations thereof. MVLs are finally obtained by removal of the volatile organic solvent from the double emulsion using gas stripping or flushing. Finally, removal of unencapsulated material, concentration of the MVLs and buffer exchange is performed using either diafiltration or cross-flow filtration system.

In one embodiment, the neutral lipids used to manufacture the MVL encapsulating eltrombopag or a conjugate or a derivative or a salt thereof comprises a mixture of triolein : tricaprylin with ratios ranging from 50:50 to 0:100.

### IMMEDIATE-RELEASE DOSAGE FORM

As mentioned above, 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof can be comprised in an immediate release dosage form.

The presence of such immediate release dosage form aims at achieving a quick medullar eltrombopag or a derivative or a conjugate or a pharmaceutically acceptable salt thereof concentration so as to obtain a rapid stabilization or increase in the local concentration SDF-1 chemokine that will in turn increase homing of transplanted HSCs. In one embodiment, the maximal weight ratio of immediate release eltrombopag or a derivative or a conjugate or a pharmaceutically acceptable salt thereof relative to the total amount of eltrombopag or a derivative or a conjugate or a pharmaceutically acceptable salt thereof is 15%.

In certain embodiment of the invention, the weight ratio of immediate release eltrombopag or a derivative or a conjugate or a pharmaceutically acceptable salt thereof relative to the total amount of eltrombopag or a derivative or a conjugate or a pharmaceutically acceptable salt thereof may be between 0 and 5%, 0 and 10%, 0 and 15%.

In some embodiments, the length of release of the immediate release form is between 0 and 6 hours. In some embodiment, the length of release of the immediate release form is between 0 and 2 hours.

The immediate release dosage form may take the form of immediate fraction that are mixed with the controlled release microparticles as described above or be present in a continuous phase to which the microparticles are mixed when preparing the final formulation under the form of a powder, according to the present invention. Said continuous phase may of course comprise any further suitable pharmaceutically acceptable excipient than the ones already present within the microparticles.

### MIXTURE OF CONTROLLED-RELEASE DOSAGE FORM AND IMMEDIATE-RELEASE DOSAGE FORM

As mentioned above, 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof can be comprised in a mixture of a controlled release dosage form and an immediate release dosage form.

The controlled release dosage form and the immediate release dosage form are those as defined above.

### PHARMACEUTICAL COMPOSITION AND KITS

As mentioned above, the pharmaceutical composition according to the invention is suitable for intramedullary injection or intramedullary implantation, that is to say for injection or implantation into the bone marrow.

Herein are provided pharmaceutical compositions in various forms, i.e. under the form of a solution, a suspension, a powder, a solid implant, a semi-solid implant, a microporous implant and an in-situ forming depot. Further pharmaceutical compositions are provided under the form of a powder or under the form of a kit. When under the form of a powder, the pharmaceutical composition is mainly aimed for storage whereas the solution, suspension, solid implant, semi-solid implant, microporous implant, powder or in-situ forming depot, in particular the suspension, is the ready to use composition, ready for injection and the kit allows to store separately (i) an aqueous injection vehicle and (ii) a controlled release dosage form comprising eltrombopag or a conjugate or a derivative or a salt thereof, in particular under the form of a powder for forming a sterile and injectable dosage form, in particular a suspension, suitable for the injection.

The pharmaceutical composition as defined in the present disclosure may comprise at least one pharmaceutically acceptable excipient in addition to 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a conjugate or a derivative or a pharmaceutically acceptable salt thereof as defined in the present disclosure.

Among the pharmaceutically acceptable excipients suitable for the pharmaceutical composition as defined in the present disclosure may be cited a tonicity-enhancing agent, a wetting-agent, a viscosity-enhancing agent, a density-enhancing agent and a mixture thereof.

In one embodiment, the pharmaceutical composition is further characterized in that it is in particular under the form of a sterile and injectable suspension, optionally comprising an excipient selected from a group consisting of a tonicity-enhancing agent, a wetting-agent, a viscosity-enhancing agent, a density-enhancing agent or a mixture thereof, and wherein it is obtained by mixing controlled release dosage form comprising eltrombopag or a conjugate or a derivative or a salt thereof, in particular microparticles comprising eltrombopag or a conjugate or a derivative or a salt thereof, more particularly as defined in the present disclosure with an aqueous injection vehicle as defined in the present disclosure.

Various embodiments of said alternative forms are detailed herein after.

Solution, solid implant, semi-solid implant, microporous implant, powder and in-situ forming depot may be prepared according to methods known to the man skilled in the art.

According to a particular embodiment, the sterile and injectable dosage form is under the form of a suspension that may be obtained from a powder, as detailed herein after.

### Powder and suspension

In one embodiment is provided a pharmaceutical composition under the form of a powder comprising a controlled release dosage form comprising eltrombopag or a conjugate or a derivative or a salt thereof, wherein said controlled release dosage form comprising eltrombopag or a conjugate or a derivative or a salt thereof is under the form of microparticles, in particular microparticles comprising a polymeric matrix, more particularly having a mean particle size equal or greater than 2 µm, and even more particularly as defined in the present disclosure.

In one embodiment is provided a pharmaceutical composition under the form of a powder comprising a controlled release microparticles comprising eltrombopag or a conjugate or a derivative or a salt thereof, wherein the microparticles are microparticles comprising a polymeric matrix or multivesicular liposomes, said microparticles comprising eltrombopag or a conjugate or a derivative or a salt thereof having a mean particle size equal or greater than 2 µm.

In another embodiment is provided a pharmaceutical composition under the form of a sterile and injectable suspension suitable for an intramedullary injection, obtained by mixing a composition under the form of a powder according to the invention, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

According to one embodiment, the powder further comprises an immediate release dosage form containing eltrombopag or a conjugate or a derivative or a salt thereof, in particular wherein the maximal weight ratio of immediate release eltrombopag or a conjugate or a derivative or a salt thereof relative to the total amount of eltrombopag or a conjugate or a derivative or a salt thereof is 15%.

In another embodiment is provided a pharmaceutical composition under the form of a powder comprising controlled release microparticles comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, and a controlled release polymeric matrix, wherein
- said controlled release microparticles have a mean particle size greater than or equal to 2 µm, in particular ranging from 2 µm to 150 µm, more particularly ranging from 10 µm to 100 µm, even more particularly ranging from 10 µm to 80 µm,
- said controlled release polymeric matrix comprises at least a poly(lactic-co-glycolic acid) copolymer, and
- the drug loading content or percentage weight ratio between the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof and the total weight of the microparticles ranges from 2% to 45%, in particular from 5% to 40%, more particularly from 10% to 35%.

According to said embodiment, the powder further comprises an immediate release dosage form containing 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a conjugate or a derivative or a pharmaceutically acceptable salt thereof, in particular EPAG olamine, in particular wherein the maximal weight ratio of immediate release 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a pharmaceutically acceptable salt thereof, in particular EPAG olamine, relative to the total amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a conjugate or a derivative or a pharmaceutically acceptable salt thereof, in particular EPAG olamine, is 15%.

The present invention further relates to a pharmaceutical composition under the form of a sterile and injectable suspension suitable for an intramedullary injection, obtained by mixing a formulation under the form of a powder as described in the present disclosure, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

In another embodiment is provided a pharmaceutical composition, in particular under the form of a sterile and injectable suspension, wherein it is obtained by mixing a controlled release dosage form, an immediate release dosage form or a mixture of a controlled release dosage form and an immediate release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a pharmaceutically acceptable salt thereof, in particular microparticles comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a conjugate or a derivative or a pharmaceutically acceptable salt thereof, more particularly as defined in the present disclosure with an aqueous injection vehicle, and wherein the pharmaceutical composition optionally comprises an excipient selected from a group consisting of a tonicity-enhancing agent, a wetting-agent, a viscosity-enhancing agent, a density-enhancing agent or a mixture thereof.

In one embodiment, said pharmaceutical composition is characterized by the concentration in eltrombopag or a conjugate or a derivative or a salt thereof ranging from 2 mg to 63 mg equivalent free acid per ml of the sterile and injectable dosage form, in particular a suspension, in particular from 8 mg to 55 mg equivalent free acid per ml, more particularly from 12 mg to 47 mg equivalent free acid per ml.

In one embodiment, said pharmaceutical composition is characterized by the concentration in eltrombopag olamine ranging from 2 mg to 80 mg per ml of the sterile and injectable dosage form, in particular a suspension, in particular from 10 mg to 70 mg per ml, more particularly from 15 mg to 60 mg per ml.

According to another particular embodiment, in a pharmaceutical composition according to the present invention still under the form of a sterile and injectable suspension, the microparticles may be present in an amount ranging from 1% to 25 % by weight, in particular from 2% to 20 % by weight, more particularly from 5 % to 15 % by weight, with respect to the total weight of the composition.

The pharmaceutical composition used in the framework of the present invention may take the form of a sterile and injectable composition, in particular a suspension composition, comprising an effective amount of eltrombopag or a conjugate or a derivative or a salt thereof.

By "sterile", in the sense of the present invention, is meant an environment capable of guaranteeing to the considered compounds in a composition according to the invention safety requirements for the administration routes such as above-mentioned, notably into or through the bone marrow. Indeed, for obvious reasons, it is essential that a composition according to the invention be devoid of any contaminant body capable of initiating an undesirable side reaction at the host site.

The pharmaceutical composition used in the framework of the present invention may be prepared with the formulation under the form of a powder comprising microparticles as described in the present disclosure. According to one embodiment, said pharmaceutical composition is a sterile and injectable composition for controlled release and/or immediate release of eltrombopag or a conjugate or a derivative or a salt thereof, suitable for an intramedullary injection.

By its injectable character, a composition according to the invention necessarily comprises a physiologically acceptable medium, also called "aqueous injection vehicle".

A "physiologically acceptable medium" means a medium devoid of toxicity and compatible with the injection and/or the application of the composition such as considered in the present invention.

The present invention more particularly relates to the pharmaceutical composition as defined in the present disclosure, wherein it is obtained by mixing a formulation under the form of a powder as describe in the present disclosure, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

The composition may comprise a solvent or a mixture of physiologically acceptable solvents.

The composition may comprise a physiologically acceptable aqueous medium.

As an aqueous medium suitable for the invention, may be for example mentioned water.

As isotonic agents suitable for the preparation of a composition according to the invention, it may be mentioned sugars and sodium chloride.

The aqueous injection vehicle may in particular comprise a tonicity-enhancing agent, a wetting-agent, a viscosity-enhancing agent or a mixture thereof.

Among the tonicity-enhancing agent, the following may be cited: dextrose, mannitol, sorbitol, sucrose, glycerin, sodium chloride, potassium chloride, cyclodextrin and maltodextrin.

Among the wetting agents, the following may be cited: poly(oxyethylene) sorbitan fatty acid esters, such as commercially available under the trade name TWEEN, sorbitan fatty acid esters, such as commercially available under the trade name SPAN, poloxamer and lecithins.

Among the viscosity-enhancing agents, the following may be cited: sodium carboxymethyl cellulose (CMC), a glycosaminoglycan such as hyaluronic acid, dextran, collagen, poly(vinylpyrrolidone), poly(ethyleneglycol), gelatin, hydroxyethyl cellulose (HEC), methyl cellulose (MC), alginate, gum acacia, starch.

According to a particular embodiment, said pharmaceutical composition has a viscosity measured at 25°C of from 5 to 1000 mPa.s, in particular from 5 to 500 mPa.s, at a shear rate of 10 s⁻¹.

### Kit

Herein is further provided a kit or article of manufacture comprising, in separate compartments (i) an aqueous injection vehicle and (ii) a controlled release dosage form, an immediate release dosage form or a mixture of a controlled release dosage form and an immediate release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a conjugate or a derivative or a pharmaceutically acceptable salt thereof, in particular as defined in the present invention or powder as defined in the present invention, wherein the pharmaceutical composition optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intramedullary injection.

In one embodiment, the kit may be under the form of two separated vials.

In another embodiment, the kit or article of manufacture may be under the form of a vial and a prefilled syringe or of a medical device or is under the form of two separated vials. In said embodiment, the two compartments or dual chamber may allow the mixture of the aqueous injection vehicle and the powder as described herein by all means known to the man skilled in the art. Still in said embodiment, said means may take the form of a pierceable membrane or destructible diaphragm, for example through a pressure the user may exert.

In these embodiments, the kit or article of manufacture may additionally contain a label instructing the user to introduce the obtained pharmaceutical composition into a subject's bone marrow.

For the comfort of the patient and for safety reasons, it is advantageous to look for a pharmaceutical composition, suitable for intramedullary injection, requiring only one effective injection thanks to this injection without needing multiple injections.

For the comfort of the patient and for safety reasons, it is also advantageous to look for a pharmaceutical composition, suitable for intramedullary implantation, requiring only one effective implantation thanks to this implantation without needing multiple implantation.

### Additional active ingredients

Depending upon the particular condition, or disease, to be treated, additional therapeutic agents, which are normally administered to treat that condition, may be administered in combination with eltrombopag or a derivative or a conjugate or a pharmaceutically acceptable salt thereof.

As used herein, the term "combination", "combined," and related terms refers to the simultaneous, separate or sequential administration of said additional active ingredient with eltrombopag or a derivative or a conjugate or a pharmaceutically acceptable salt thereof. For example, a combination may be administered with an additional active ingredient simultaneously, separately or sequentially in separate unit dosage forms or together in a single unit dosage form.

In some embodiments, the present invention provides a pharmaceutical composition comprising eltrombopag or a derivative or a conjugate or a pharmaceutically acceptable salt thereof according to the present invention further comprising at least an additional therapeutic agent. Suitable additional active ingredients are described in further detail below.

In a particular embodiment, the patient is simultaneously, separately or sequentially treated by at least one active ingredient to prevent Graft-versus-host disease, said at least one active ingredient being selected from cyclosporine A, methotrexate, tacrolimus, mycophenolate mofetil, anti-thymocyte globulin serum, cyclophosphamide, and abatacept.

In a particular embodiment, the patient is simultaneously, separately or sequentially treated by at least one active ingredient selected from thrombopoietin (TPO) receptor agonist distinct from eltrombopag or a derivative or a conjugate or a pharmaceutically acceptable salt thereof. Such thrombopoietin (TPO) receptor agonists can be selected from thrombopoietin, romiplostim, avatrombopag, and hetrombopag.

The additional active ingredients suitable for the present disclosure can be administered simultaneously, separately or sequentially *via* any route of administration, that is to say for example *via* oral route or parenteral route.

Said additional active ingredient may be formulated under immediate and/or controlled release dosage forms.

### Additional therapies

Depending on the patient to be treated, the patient can be treated prior to or after the intramedullary administration of the pharmaceutical composition as defined in the present disclosure or as obtained by mixing the two compartments of the kit as defined in the present disclosure, with at least one therapy selected from radiation therapy, radiomimetic chemotherapy and mixtures thereof.

In a particular embodiment, the radiomimetic chemotherapy can be selected from the group consisting of ozone, peroxide, an alkylating agent, a platinum-based agent, a cytotoxic antibiotic, and a vesicant chemotherapy, preferably, the radiomimetic chemotherapy is cyclophosphamide, busulfan, fludarabine, melphalan, thiotepa, cytarabine and clofarabine, carmustine, etoposide, cytarabine and melphalan, Rituximab, ifosfamide, etoposide, or a platinum- based agent selected from the group consisting of cisplatin, carboplatin, oxaliplatin, and nedaplatin.

### Administration of the composition

Pharmaceutical composition according to the invention can be injected or implanted in any suitable site containing functioning bone marrow like in the sternum, tibia, femur, iliac crest or vertebra, in particular in the iliac crest, more particularly in the posterior part of the iliac crest.

Intramedullary injection can be performed using a 18G needle equipped with a trocar.

Intramedullary implantation can be performed using a 16G needle equipped with a trocar.

Injection or implantation can be performed under general or locoregional anesthesia.

A pharmaceutical composition used in the framework of the present invention can be injected or implanted using any of the known methods in the art.

Particularly, said pharmaceutical composition may be administered by means of an injection device suitable for intramedullary injection such as a syringe equipped with a needle 15-25G, preferentially 16-25G, more preferentially 18-23G.

### THERAPEUTIC USES AND METHODS

As mentioned above, according to one embodiment, the pharmaceutical composition as defined in the present disclosure or as obtained by mixing the two compartments of the kit as defined in the present disclosure is intramedullary injected or intramedullary implanted once or several times in a patient prior to or during or after an autologous or allogeneic hematopoietic stem cell transplantation (HSCT), in particular a human patient, and is useful for improving the homing, engraftment and long - term expansion and proliferation of hematopoietic stem cells.

According to a particular embodiment, herein is provided a pharmaceutical composition as defined in the present disclosure or as obtained by mixing the two compartments of the kit as defined in the present disclosure, for use for improving the homing, engraftment and long - term expansion and proliferation of hematopoietic stem cells by using an implant in a patient prior to or during or shortly after an autologous or allogeneic hematopoietic stem cell transplantation (HSCT), in particular a human patient.

The hematopoietic stem cells may be selected from bone marrow cells, peripheral blood stem cells, cord blood cells cultured stem cells, including cultured stem cells after gene therapy.

The pharmaceutical composition as defined in the present disclosure may be injected or implanted in the bone marrow within 96 hours prior to and 96 hours after transplantation, in particular within 96 hours prior to and 24 hours after transplantation, more particularly within 96 hours prior to transplantation.

More particularly, the pharmaceutical composition as defined in the present disclosure may be useful for decreasing graft failure, for decreasing the occurrence of Poor Graft function (PGF), for decreasing the occurrence of Graft-versus-host disease (GvHD), for enhancing overall survival and donor chimerism, and/or for promoting hematopoietic recovery and hematopoietic stem cell transplantation success rate.

According to a particular embodiment, herein is provided a pharmaceutical composition as defined in the present disclosure or as obtained by mixing the two compartments of the kit as defined in the present disclosure, wherein the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a conjugate or a derivative or a pharmaceutically acceptable salt thereof, in particular EPAG olamine, is administered by intramedullary injection into said patient in an amount ranging from 0,2 mg to 50 mg equivalent free acid per kg of body weight of said patient, in particular ranging from 0,5 mg to 45 mg equivalent free acid per kg of body weight of said patient, more particularly from 1 mg to 40 mg equivalent free acid per kg of body weight of said patient.

According to another embodiment, the pharmaceutical as defined in the present disclosure or as obtained by mixing the two compartments of the kit as defined in the present disclosure is used in the prevention and/or treatment of non-malignant blood disorders such as severe aplastic anemia, and hemoglobinopathies, in particular Sickle cell disease and beta-Thalassemia and/or in the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia and/or in the prevention and/or treatment of primary immunodeficiencies, autoimmune diseases or inborn errors of metabolism.

The load dose of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a conjugate or a derivative or a pharmaceutically acceptable salt thereof, in particular EPAG olamine, is an amount ranging from 12 mg to 725 mg administered intramedullary in the bone marrow within 96 hours prior to and 96 hours after transplantation, in particular within 96 hours prior to and 24 hours after transplantation, more particularly within 96 hours prior to transplantation.

Further, the time required to release 80% by weight of the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a conjugate or a derivative or a pharmaceutically acceptable salt thereof, in particular EPAG olamine, is greater than 4 day and may reach 30 days preferably 15 days, wherein the 3'-[(2Z)-[1-(3,4-dimethylphenyl)-1,5-dihydro-3-methyl-5-oxo-4H-pyrazol-4-ylidene]hydrazino]-2'-hydroxy-[1,1 '-biphenyl]-3-carboxylic acid or a conjugate or a derivative or a pharmaceutically acceptable salt thereof, in particular EPAG olamine, is present at a dose ranging from 10 mg to 570 mg. equivalent to Eltrombopag free acid.

Further, the time required to release 80% by weight of EPAG olamine, is greater than 4 days and may reach 30 days preferably 15 days, wherein the EPAG olamine, is present at a dose ranging from 12 mg to 725 mg.

As above mentioned, the controlled release dosage form, the immediate release dosage form or the mixture of a controlled release dosage form and an immediate release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof as defined in the present disclosure, is administered locally by injection or implantation for improving the homing, engraftment and long - term expansion and proliferation of hematopoietic stem cells in a patient prior to or during or after an autologous or allogeneic hematopoietic stem cell transplantation (HSCT), and/or in the prevention and/or treatment of non-malignant blood disorders such as severe aplastic anemia, and hemoglobinopathies, in particular Sickle cell disease and beta-Thalassemia and/or in the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia and/or in the prevention and/or treatment of primary immunodeficiencies, autoimmune diseases or inborn errors of metabolism. Local administration of the formulation may occur by injection or implantation into the bone marrow.

In one embodiment, the pharmaceutical composition suitable for an intramedullary injection or intramedullary implantation, is dedicated to the improving of the homing of hematopoietic stem cells prior to or during or after an autologous or allogeneic hematopoietic stem cell transplantation (HSCT).

In one embodiment, the composition suitable for an intramedullary injection or intramedullary implantation, is dedicated to the improving of the engraftment of hematopoietic stem cells prior to or during or after an autologous or allogeneic hematopoietic stem cell transplantation (HSCT).

In one embodiment, the composition suitable for an intramedullary injection or intramedullary implantation, is dedicated to the improving of the long - term expansion and proliferation of hematopoietic stem cells prior to or during or after an autologous or allogeneic hematopoietic stem cell transplantation (HSCT).

In one embodiment, the pharmaceutical composition suitable for an intramedullary injection or intramedullary implantation, is dedicated to the prevention and/or treatment of non-malignant blood disorders such as severe aplastic anemia, and hemoglobinopathies, in particular Sickle cell disease and beta-Thalassemia.

In one embodiment, the pharmaceutical composition suitable for an intramedullary injection or intramedullary implantation, is dedicated to the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia.

In one embodiment, the pharmaceutical composition suitable for an intramedullary injection or intramedullary implantation, is dedicated to the prevention and/or treatment of primary immunodeficiencies, autoimmune diseases or inborn errors of metabolism.

In one embodiment, the pharmaceutical composition suitable for an intramedullary injection or intramedullary implantation, is dedicated to the prevention and/or treatment of severe aplastic anemia.

In one embodiment, the pharmaceutical composition suitable for an intramedullary injection or intramedullary implantation, is dedicated to the prevention and/or treatment of Sickle cell disease.

In one embodiment, the pharmaceutical composition suitable for an intramedullary injection or intramedullary implantation, is dedicated to the prevention and/or treatment of beta-Thalassemia.

In one embodiment, the pharmaceutical composition suitable for an intramedullary injection or intramedullary implantation, is dedicated to the prevention and/or treatment of myeloma and/or lymphoma.

In one embodiment, the pharmaceutical composition suitable for an intramedullary injection or intramedullary implantation, is dedicated to the prevention and/or treatment of leukemia, such as acute myeloid leukemia, acute lymphoblastic leukemia, chronic lymphocytic leukemia, and chronic myeloid leukemia.

In one embodiment, the pharmaceutical composition suitable for an intramedullary injection or intramedullary implantation, is dedicated to the prevention and/or treatment of Waldenstrom macroglobulinemia.

In one embodiment, the pharmaceutical composition suitable for an intramedullary injection or intramedullary implantation, is dedicated to the prevention and/or treatment of myeloproliferative neoplasms.

In one embodiment, the pharmaceutical composition suitable for an intramedullary injection or intramedullary implantation, is dedicated to the prevention and/or treatment of myelodysplastic syndrome.

The administration may be performed by a single injection or as sequential injections provided that the time required to release 80% by weight of the eltrombopag or a derivative or a conjugate or a pharmaceutically acceptable salt thereof, in particular EPAG olamine, in the microparticles is greater than 4 day and may reach 30 days, in particular 15 days, the eltrombopag or a derivative or a conjugate or a pharmaceutically acceptable salt thereof, in particular EPAG olamine, being present in a concentration ranging from 2 mg to 80 mg per ml of suspension and the pharmaceutical composition having a volume ranging from 5 ml to 10 ml. In other words, the single or sequential injections, preferably the single injection, provides locally an eltrombopag olamine weight, ranging between 12 mg and 725 mg.

In one embodiment, the injection is performed a plurality of times (two times, three times, four times or more). In this embodiment, each subsequent time the injection is performed, it is performed after a suitable period has lapsed since the preceding time the injection was performed. This suitable time can be, for example, two weeks, one month, two months, three months, four months, five months, six months, one year, or longer.

According to a particular embodiment, the eltrombopag or a derivative or a conjugate or a pharmaceutically acceptable salt thereof, in particular EPAG olamine, is present in a concentration ranging from 2 mg to 63 mg equivalent free acid per ml of suspension, in particular from 8 mg to 55 mg equivalent free acid per ml of suspension, and more particularly from 12 mg to 47 mg equivalent free acid per ml of suspension.

According to a particular embodiment, the eltrombopag olamine is present in a concentration ranging from 2 mg to 80 mg per ml of suspension, in particular from 10 mg to 70 mg per ml of suspension, and more particularly from 15 mg to 60 mg per ml of suspension.

In one embodiment, the pharmaceutical composition for use according to the invention, is characterized by the fact that the time required to release 80% by weight of the eltrombopag or a derivative or a conjugate or a pharmaceutically acceptable salt thereof, in particular EPAG olamine, in the microparticles is greater than 4 days, for example 4.5 days, and may reach 30 days, is greater than 4 days, for example 4.5 days, and may reach 20 days, is greater than 4 days, for example 4.5 days, and may reach 15 days, and for example is greater than 4 days, for example 4.5 day, 5 days, 6 days, 7 days, 8 days, 9 days, 10 days, 11 days, 12 days, 13 days, 14 days or 15 days, more particularly is greater than 4 days, for example 4.5 days, and may reach 7 days, or is comprised between 5 days and 15 days, between 5 days and 10 days and between 5 days and 7 days.

The pharmaceutical composition may or may not exhibit a burst release. In the framework of the present invention a "burst release" means that an initial bolus of drug is released before the release rate reaches a stable profile, immediately upon placement in the release medium, i.e. in the present invention immediately after the intramedullary injection.

In any case, the length of burst release, may be between 0 and 1 day, for example between the beginning of day 1 through the end of day 1, and in particular is between 0 and 6 hours, between 0 and 3 hours, and even more particularly is between 0 and 2 hours.

In a particular embodiment, the pharmaceutical composition for use according to the present invention does not exhibit an important burst release. According to said embodiment, less than 50% by weight of eltrombopag or a derivative or a conjugate or a pharmaceutically acceptable salt thereof is released at 12 hours after the administration.

According to another aspect, the invention relates to a method for improving the homing, engraftment and long - term expansion and proliferation of hematopoietic stem cells prior to or during or after an autologous or allogeneic hematopoietic stem cell transplantation (HSCT), comprising at least the administering by intramedullary injection to a patient in need thereof, in particular a human patient in need thereof, of a pharmaceutical composition under the form of a suspension comprising a controlled-release polymeric matrix and an effective amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, suitable for intramedullary injection.

According to another aspect, the invention relates to a method for decreasing graft failure, for decreasing graft failure, for decreasing the occurrence of Poor Graft function (PGF), for decreasing the occurrence of Graft-versus-host disease (GvHD), for enhancing overall survival and donor chimerism, and/or for promoting hematopoietic recovery and hematopoietic stem cell transplantation success rate comprising at least the administering by intramedullary injection to a patient in need thereof, in particular a human patient in need thereof, of a pharmaceutical composition under the form of a suspension comprising a controlled-release polymeric matrix and an effective amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, suitable for intramedullary injection.

According to another aspect, the invention relates to a method for the prevention and/or treatment of non-malignant blood disorders such as severe aplastic anemia, and hemoglobinopathies, in particular Sickle cell disease and beta-Thalassemia comprising at least the administering by intramedullary injection to a patient in need thereof, in particular a human patient in need thereof, of a pharmaceutical composition under the form of a suspension comprising a controlled-release polymeric matrix and an effective amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, suitable for intramedullary injection.

According to another aspect, the invention relates to a method for the prevention and/or treatment of malignant hemopathies such as myeloma, lymphoma, in particular selected from leukemia, such as acute myeloid leukemia, acute lymphoblastic leukemia, chronic lymphocytic leukemia, chronic myeloid leukemia, myeloma, Waldenstrom macroglobulinemia, myeloproliferative neoplasms, myelodysplastic syndrome comprising at least the administering by intramedullary injection to a patient in need thereof, in particular a human patient in need thereof, of a pharmaceutical composition under the form of a suspension comprising a controlled-release polymeric matrix and an effective amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, suitable for intramedullary injection.

According to another aspect, the invention relates to a method for the prevention and/or treatment of primary immunodeficiencies, autoimmune diseases or inborn errors of metabolism comprising at least the administering by intramedullary injection to a patient in need thereof, in particular a human patient in need thereof, of a pharmaceutical composition under the form of a suspension comprising a controlled-release polymeric matrix and an effective amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, suitable for intramedullary injection.

According to another aspect, the invention relates to a method for improving the homing, engraftment and long - term expansion and proliferation of hematopoietic stem cells prior to or during or after an autologous or allogeneic hematopoietic stem cell transplantation (HSCT), comprising at least the administering by intramedullary injection or by intramedullary implantation to a patient in need thereof, in particular a human patient in need thereof, of at least an efficient amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof for a controlled release and/or an immediate release.

According to another aspect, the invention relates to a method for decreasing graft failure, for decreasing the occurrence of Poor Graft function (PGF), for decreasing the occurrence of Graft-versus-host disease (GvHD), for enhancing overall survival and donor chimerism, and/or for promoting hematopoietic recovery and hematopoietic stem cell transplantation success rate comprising at least the administering by intramedullary injection or intramedullary implantation to a patient in need thereof, in particular a human patient in need thereof, of at least an efficient amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof for a controlled release and/or an immediate release.

According to another aspect, the invention relates to a method for the prevention and/or treatment of non-malignant blood disorders such as severe aplastic anemia, and hemoglobinopathies, in particular Sickle cell disease and beta-Thalassemia comprising at least the administering by intramedullary injection or intramedullary implantation to a patient in need thereof, in particular a human patient in need thereof, of at least an efficient amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof for a controlled release and/or an immediate release.

According to another aspect, the invention relates to a method for the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia comprising at least the administering by intramedullary injection or intramedullary implantation to a patient in need thereof, in particular a human patient in need thereof, of at least an efficient amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof for a controlled release and/or an immediate release.

According to another aspect, the invention relates to a method for the prevention and/or treatment of primary immunodeficiencies, autoimmune diseases or inborn errors of metabolism comprising at least the administering by intramedullary injection or intramedullary implantation into a patient in need thereof, in particular a human patient in need thereof, of at least an efficient amount of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof for a controlled release and/or an immediate release.

Herein is further provided a method for improving the homing, engraftment and long - term expansion and proliferation of hematopoietic stem cells prior to or during or after an autologous or allogeneic hematopoietic stem cell transplantation (HSCT), and/or for decreasing graft failure, for decreasing the occurrence of Poor Graft function (PGF), for decreasing the occurrence of Graft-versus-host disease (GvHD), for enhancing overall survival and donor chimerism, and/or for promoting hematopoietic recovery and hematopoietic stem cell transplantation success rate, and/or for the prevention and/or treatment of non-malignant blood disorders such as severe aplastic anemia, and hemoglobinopathies, in particular Sickle cell disease and beta-Thalassemia and/or for the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia and/or for the prevention and/or treatment of primary immunodeficiencies, autoimmune diseases or inborn errors of metabolism comprising at least the steps of:
- preparing and/or providing a pharmaceutical composition under the form of a sterile and injectable suspension by mixing a formulation under the form of a powder as described herein after, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder, and
- injecting intramedullary said pharmaceutical composition into bone marrow of a patient in need thereof, wherein the volume to be injected is adapted to the injection site.

Herein is further provided a method for improving the homing, engraftment and long - term expansion and proliferation of hematopoietic stem cells prior to or during or after an autologous or allogeneic hematopoietic stem cell transplantation (HSCT), and/or for decreasing graft failure, for decreasing the occurrence of Poor Graft function (PGF), for decreasing the occurrence of Graft-versus-host disease (GvHD), for enhancing overall survival and donor chimerism, and/or for promoting hematopoietic recovery and hematopoietic stem cell transplantation success rate, and/or for the prevention and/or treatment of non-malignant blood disorders such as severe aplastic anemia, and hemoglobinopathies, in particular Sickle cell disease and beta-Thalassemia and/or for the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia and/or for the prevention and/or treatment of primary immunodeficiencies, autoimmune diseases or inborn errors of metabolism comprising at least the steps of:
- preparing and/or providing a pharmaceutical composition under the form of a sterile and injectable solid implant, semi-solid implant, microporous implant or in situ forming depot, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder, and
- implanting intramedullary said pharmaceutical composition into bone marrow of a patient in need thereof, wherein the volume to be implanted is adapted to the implantation site.

In one embodiment, the pharmaceutical composition for use according to the present invention may further be characterized by the fact that the patient to which said pharmaceutical composition is administered may simultaneously, separately or sequentially be treated by the following active ingredients: cyclosporine A, methotrexate, tacrolimus, mycophenolate mofetil, anti-thymocyte globulin serum, cyclophosphamide, abatacept, thrombopoietin receptor agonists selected from thrombopoietin, romiplostim, avatrombopag, and hetrombopag, and mixtures thereof.

Herein is thus provided the pharmaceutical composition for use as defined in the present disclosure wherein it is for use in the prevention and/or treatment of non-malignant blood disorders such as severe aplastic anemia, and hemoglobinopathies, in particular Sickle cell disease and beta-Thalassemia and/or in the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia and/or in the prevention and/or treatment of primary immunodeficiencies, autoimmune diseases or inborn errors of metabolism, in a patient being simultaneously, separately or sequentially treated by at least one of the active ingredient selected from: cyclosporine A, methotrexate, tacrolimus, mycophenolate mofetil, anti-thymocyte globulin serum, cyclophosphamide, abatacept, thrombopoietin receptor agonists selected from thrombopoietin, romiplostim, avatrombopag, and hetrombopag, and mixtures thereof.

It should also be understood that a specific dosage and treatment regimen for any particular patient will depend upon a variety of factors, including the age, body weight, general health, sex, diet, time of administration, drug combination, and the judgment of the treating physician and the severity of the particular disease being treated.

Throughout the description, including the claims, the expression "comprising a" should be understood as being synonymous with "comprising at least one" unless specifically stated otherwise.

The expressions "between... and ..." and "ranging from ... to ..." should be understood to mean that the limits are inclusive, unless specified otherwise.

The following examples and figures are presented by way of non-limiting illustration of the invention.

### EXAMPLES

### Analytical methods

- Particle size was determined using a Malvern Mastersizer MS3000 laser diffraction particle size analyzer. A 1 mg/ml microparticles in deionized water preparation was introduced in the apparatus and the measurement run 3 times. The results shown in the examples are the mean of the three measures.
- Eltrombopag microparticle drug load was determined by first solubilizing 10 mg of drug-loaded polymeric microparticles in 2,5 mL of an acetonitrile/water mixture at a 80/20 v/v ratio. The medium was then centrifuged for 15 min at 4000 rpm and the supernatant filtrated through a 0.45 µm PTFE filter (Acrodisc PTFE). A 2 ml aliquot of the filtrated supernatant was then analyzed by HPLC to determine the microparticle drug load.
   HPLC analysis was conducted using a silica-based reversed-phase C18 column [Kinetex C18, 100mm × 4,6 mm, 2,6 µm particle size] and a mobile phase consisting in [acetonitrile/ammonium formate (10 mM) 80/20 v/v adjusted at pH 2]. The flow rate was set at 0,5 mL/min, the injection volume was 10µL and the UV detection was set at 244 nm.
- For incubation of Eltrombopag olamine with activated human proteases, proMMP-8 and pro MMP-9 proteases were first activated. Human proMMP-8 (Cat. no. 908-MP, R&D systems) at 100 µg/ml in buffer containing 50 mM Tris, 150 mM NaCl (sodium chloride), 5 mM CaCl₂ (calcium chloride) and 0.01% tween 20 (pH 7.6) was activated by incubation with 1 mM p-aminophenylmercuric acetate for 1h at 37°C. Human proMMP-9 was expressed in Sf9 cells, purified by gelatin-sepharose chromatography and activated by incubation with the catalytic domain of MMP-3 (cat. No. 444217, Merck Millipore). Specifically, human proMMP-9 at 920 µg/ml in the same described buffer as for MMP-8 activation was incubated with the catalytic domain of MMP-3 at a molar ratio of 1/100 (MMP-3/MMP-9) for 2h at 37°C in. The final concentration of MMP-9 was 10 µM and the final concentration of MMP-3 was 0.1 µM. Concentrated eltrombopag olamine solution was prepared by solubilization in water at a concentration of 10 mg/ml for 24h on rotor at 4°C, and further diluted at the final concentration needed. Activated MMP-8 or MMP-9 were then incubated at a final concentration of 625 nM in Eltrombopag olamine at different concentrations (50 µM, 10 µM, 1 µM and 0.1 µM) in a final volume of 3µl for 30 minutes at 37°C in calcium-free buffer containing 50 mM Tris, 150 mM NaCl, 0.01% tween 20 (pH 7.6) using low protein-binding tubes (Protein LoBind tubes, Eppendorf).
- Incubation of human SDF-1α (Cat. no. 300-28A, Peprotech) in the solution containing the activated human proteases and eltrombopag olamine was done at a MMP/SDF-1 molar ratio of 1/5 in the same calcium-free buffer and tubes described, with a final SDF-1 α concentration of 3.125 µM and a final volume of 4µl at 37°C for 2h. A negative control without eltrombopag olamine was similarly prepared.
- SDF-1 proteolysis was analyzed by multiplex western blot. All samples were diluted in reducing loading buffer (10% β-mercaptoethanol, 0.1% bromophenol blue, 4% SDS (sodium dodecyl sulfate), 20% glycerol, 125 mM Tris, pH 6.8) and boiled at 90°C for 20 min. Next, the equivalent of 100 ng SDF-1 was loaded onto Novex 16% tricine gels (Cat. no. EC6695BOX, Invitrogen). The Chameleon Duo Pre-stained Protein Ladder was used as a molecular weight using 6ul in one well. Proteins in the samples were separated by denaturing SDS-PAGE using tricine running buffer (100 mM Tris, 100 mM Tricine, 0.1% SDS, pH 8.3) under a constant voltage of 120V (±60 min). After electrophoresis, proteins were transferred to polyvinylidene difluoride (PVDF) membranes with the Trans-Blot Turbo Transfer System (Biorad) with Trans-Blot Turbo RTA Mini 0.45 µm low fluorescence PVDF Transfer Kit (Cat. No. 1704274, Biorad). After blocking the membranes with Intercept (TBS, Tris-buffered Saline) blocking buffer (Cat. no. 927-60001, Licor) for 60 min at room temperature, the membranes were incubated overnight at 4°C on a rotatory shaker with a rabbit polyclonal primary antibody recognizing all SDF-1 isoforms (cell Signaling 3740S, 1:1000) and a mouse monoclonal antibody recognizing the N-terminus SDF-1 part typically cleaved by protease activity causing suppression of SDF-1 chemotactic function (Millipore MABC184, 1:2000) in TBS blocking buffer with 0.2% Tween 20. After three washes of 10 min in Tris buffered saline with Tween 20 (150 mM NaCl, 20 mM Tris, 0.1% tween 20), incubation with the secondary antibodies donkey anti-rabbit 680 RD (Cat. no. 926-68073, Licor, 1:5000) and donkey anti-mouse 800 CW (Cat. no. 926-32212, Licor, 1:5000), diluted in TBS blocking buffer with 0.2% Tween 20 and 0.02% SDS, was performed for 1h at room temperature on a rotary shaker. After three washes of 10 min in Tris buffered saline with Tween 20, the membranes were imaged using the Odyssey Fc Imaging System (LiCor). Protein bands quantification was done using the Empiria Studio Software (Licor) expressing the ratio of intact SDF-1 signal in relative fluorescence units (RFUs) per total (all isoforms) SDF-1 RFUs.

### Example 1: Sustained-release Eltrombopag olamine microparticles (S/O/W emulsion

Loading of Eltrombopag olamine into PLGA microparticles was performed using the solid-in-oil-in-water (S/O/W) emulsion and solvent evaporation process.

In this experiment, Eltrombopag olamine, supplied as a powder with an average particle diameter (D50) of 3 µm is used.

4,16 g of PLGA 50:50 Resomer 503H and 0,21 g of lecithin (Phospholipon 90H, Lipoid) were first solubilized in 14,3 g of methylene chloride under magnetic stirring during 1 hour. 2 g of Eltrombopag olamine powder was then dispersed in this organic solvent using high shear rotor/stator mixing (IKA T18 ultra-Turrax) at 10,000 rpm for 1 minute.

This S/O dispersion was then emulsified by mixing with 26,3 mL of a 1 wt % PVA aqueous solution (Mowiol 4-88, Sigma-Aldrich) at 9500 rpm using an IKA T18 ultra-Turrax during 10 min.

The emulsion was then rapidly poured into an extraction bath (3 L of an aqueous PVA solution at 1%) and stirred for 3 hours at 300 rpm with an overhead stirrer.

The solid particles were then separated from aqueous phase by centrifugation at 4000 rpm during 4 min, filtered on a 40 µm stainless steel sieve and rinsed three times 50 mL of distilled water.

The washed microparticles were stored in a glass overnight and then dried under vacuum at 5 mBar for 18 hours.

The collected microparticles were finally stored at 5°C.

**Table 1. Analytical results Eltrombopag olamine PLGA microparticles**

| Drug loading content (Eltrombopag olamine wt %) | Particle size (Dv, µm) |
|---|---|
| 26% | D10 = 2.8 µm |
| | D50 = 6.9 µm |
| | D90 = 17.8 µm |

In this example, Eltrombopag olamine-loaded PLGA microparticles were prepared with a mean particle size of 6,9 µm and a drug loading content of 26 wt %.

### Example 2: Pharmaceutical composition - Preparation of injectable suspension

An aqueous injection vehicle was prepared using pyrogen-free excipients and consisting of 1.4% of low viscosity sodium carboxymethyl cellulose (Aqualon CMC 7LF PH BET, Ashland), 0.1% Polysorbate 20 (Acros Organics), 0.13% disodium hydrogen phosphate dihydrate, 0.1% citric acid (Roth) and 0.65% sodium chloride (Roth). Final pH of the solution was adjusted to 7.2 using concentrated sodium hydroxide solution (Roth). The vehicle was then autoclaved at 121°C for 15 minutes (MultiControl 2, CertoClav) and 5 mL aliquots of the solution transferred aseptically into 10 mL vials under a laminar air flow bench.

A 470 mg aliquot of microparticles as prepared according to example 1 was dispersed in each vial using a vortex mixer

This pharmaceutical composition is intended for intra-medullary injection prior to allogenic or autologous HSCT for the treatment of malignant and non-malignant hemopathies such as severe aplastic anemia, Sickle cell disease, beta-Thalassemia, myeloma, lymphoma, acute myeloid leukemia, acute lymphoblastic leukemia, chronic lymphocytic leukemia, chronic myeloid leukemia, Waldenstrom macroglobulinemia, myeloproliferative neoplasms, myelodysplastic syndrome, primary immunodeficiencies, autoimmune diseases or inborn errors of metabolism.

In an example, a volume of 5 mL containing a 123 mg eltrombopag olamine dose can be injected into the posterior iliac crest using a 18G needle equipped with a trocar.

### Example 3: Sustained-release Eltrombopag olamine microparticles (W/O/W emulsion)

Loading of Eltrombopag olamine into PLGA microparticles was performed using the water-in-oil-in-water (W1/O/W2) double emulsion and solvent evaporation process.

An internal water phase W1 was obtained by solubilizing 20 mg of Eltrombopag olamine (MedChemExpress, USA) and 50 mg of 2-hydroxypropyl-β-cyclodextrin (Sigma Aldrich) in 1mL of water.

An organic solvent phase was prepared by solubilizing 500 mg of PLGA Resomer RG 503H (Evonik Industries AG, Essen, Germany) and 25 mg of lecithin (Phospholipon 90H, Lipoid) in 2,5 mL of dichloromethane.

For preparation of the W1/O primary emulsion, the W1 phase and oil phase were emulsified under high shear rotor/stator mixing at 24000 rpm during 10 min.

W1/O/W2 double emulsion was then obtained by mixing W1/O in 1 mL of water containing PVA (Mowiol 4-88, Sigma-Aldrich) at 1% and emulsifying under high shear rotor/stator mixing at 9500 rpm during 5 min

Double emulsion was then poured into an extraction bath (0,5 L of an aqueous PVA solution at 1%) and stirred for 3 hours at 300 rpm with an overhead stirrer.

The solid particles were then separated from aqueous phase by centrifugation at 4000 rpm during 4 min and finally dried at 15°C under 5 mbar pressure for 18 hours.

**Table 2. Analytical results Eltrombopag olamine PLGA microparticles**

| Drug loading content (Eltrombopag olamine wt %) | Particle size (Dv, µm) |
|---|---|
| 2.3% | D10 = 2.8 µm |
| | D50 = 5.1 µm |
| | D90 = 8.6 µm |

### Example 4: SDF-1 stabilization by eltrombopag olamine-mediated MMP-9 protease inhibition

Activated human MMP-9 was incubated with different eltrombopag olamine concentrations (50 uM, 10 uM, 1 uM and 0.1 uM) at 37°C for 1h. Subsequently, human SDF-1α was added to the already prepared solution at a MMP/SDF-1α molar ratio of 1/5 at 37°C for 2h. Subsequently, proteolysis of SDF-1α was analysed by multiplex Western blot by detecting total SDF-1α and protease-inactivated SDF-1α. Results clearly showed that 50µM Eltrombopag olamine is able to strongly reduce the proteolytic effect of MMP-9 on SDF-1α (72% of intact SDF-1α is retrieved when MMP-9 is in the presence of 50 µM eltrombopag olamine, in comparison to only 20% in the absence of eltrombopag olamine; Figures 1A and 1B and Table 3).

**Table 3: Inhibitory effect of eltrombopag olamine on the proteolysis of SDF-1 by MMP-9.**

| **Lane content** | **N-term intact SDF-1α (RFUs)** | **Total SDF-1α (RFUs)** | **Ratio of intact/total SDF-1α** |
|---|---|---|---|
| Lane 1: SDF-1 | 0,046 | 4,278 | 0,011 |
| Lane 2: SDF-1 plus MMP-9 | 0,009 | 3,480 | 0,003 |
| Lane 3: SDF-1 plus MMP-9 plus 50 µM eltrombopag olamine | 0,033 | 4,687 | 0,007 |
| Lane 4: SDF-1 plus MMP-9 plus 10 µM eltrombopag olamine | 0,010 | 4,142 | 0,002 |
| Lane 5: SDF-1 plus MMP-9 plus 1 µM eltrombopag olamine | 0,012 | 3,644 | 0,003 |
| Lane 6: SDF-1 plus MMP-9 plus 0.1 µM eltrombopag olamine | 0,013 | 3,951 | 0,003 |

### Example 5: SDF-1 stabilization by eltrombopag olamine-mediated MMP-8 protease inhibition

Activated human MMP-8 was incubated with different eltrombopag olamine concentrations (50 µM, 10 µM, 1 µM and 0.1 µM) at 37°C for 1h. Subsequently, human SDF-1α was added to the already prepared solution at a MMP/SDF-1α molar ratio of 1/5 at 37°C for 2h. Subsequently, proteolysis of SDF-1α was analysed by multiplex Western blot by detecting total SDF-1α and protease-inactivated SDF-1α. Results clearly showed that 50µM Eltrombopag olamine is able to reduce the proteolytic effect of MMP-8 on SDF-1α (30% of intact SDF-1α is retrieved when MMP-8 is in the presence of 50 µM eltrombopag olamine, in comparison to only 3.5% in the absence of eltrombopag olamine; Figures 2A and 2B and Table 4).

**Table 4: Inhibitory effect of eltrombopag olamine on the proteolysis of SDF-1 by MMP-8.**

| **Lane content** | **N-term intact SDF-1α (RFUs)** | **Total SDF-1α (RFUs)** | **Ratio of intact / total SDF-1α** |
|---|---|---|---|
| Lane 1: SDF-1 | 0,0069 | 14,856 | 0,0005 |
| Lane 2: SDF-1 plus MMP-8 | 0,0002 | 9,119 | 0,0000 |
| Lane 3: SDF-1 plus MMP-8 plus 50 µM eltrombopag olamine | 0,0020 | 10,430 | 0,0002 |
| Lane 4: SDF-1 plus MMP-8 plus 10 µM eltrombopag olamine | 0,0001 | 6,989 | 0,0000 |
| Lane 5: SDF-1 plus MMP-8 plus 1 µM eltrombopag olamine | 0,0001 | 7,986 | 0,0000 |
| Lane 6: SDF-1 plus MMP-8 plus 0.1 µM eltrombopag olamine | 0,0006 | 7,501 | 0,0001 |

## Claims

1. A pharmaceutical composition comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl] - 3-carboxylic acid or a derivative or a conjugate or a pharmaceutically acceptable salt thereof, wherein it is suitable for intramedullary injection or intramedullary implantation.

2. The pharmaceutical composition according to claim 1, wherein the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof is under the form of bis-monoethanolamine salt of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid.

3. The pharmaceutical composition according to claim 1 or 2, wherein it comprises a controlled release dosage form, an immediate release dosage form or a mixture of a controlled release dosage form and an immediate release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof.

4. The pharmaceutical composition according to claim 3, wherein the controlled release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof is under the form of an in-situ forming depot, a hydrogel, a microporous implant, a solid implant or microparticles, in particular microparticles comprising a controlled-release polymeric matrix or multivesicular liposomes.

5. The pharmaceutical composition according to claim 3 or claim 4, wherein the controlled release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof is under the form of microparticles, in particular microparticles comprising a controlled-release polymeric matrix, and wherein said microparticles have a mean particle size equal or greater than 2 µm, and in particular ranging from 2 µm to 150 µm, more particularly from 10 µm to 100 µm, more preferably from 10 µm to 80 µm.

6. The pharmaceutical composition according to claim 4 or claim 5, wherein the controlled-release polymeric matrix comprises at least one biocompatible and biodegradable copolymer or polymer selected from a poly(lactic-co-glycolic acid) copolymer, poly(caprolactone), poly(lactide), poly(glycolide), poly(lactide-co-caprolactone), poly(ethylene glycol), poly(ethylene oxide), PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol), PVA-g-PLGA, poly (ether ester) multiblock copolymers, polyvinyl pyrrolidone, poly(methacrylate), PEO-PPO-PEO, gelatin, heparin, chondroitin sulfate; polysaccharides such as alginates, starch, chitosan, hyaluronic acid and dextrans, and any combinations thereof.

7. The pharmaceutical composition according to any one of claims 4 to 6, wherein the controlled-release polymeric matrix comprises at least a poly(lactic-co-glycolic acid) copolymer.

8. The pharmaceutical composition according to any one of claims 4 to 7, wherein the controlled-release polymeric matrix comprises poly(lactic-co-glycolic acid), in an amount of greater than 70 % by weight, with respect to the total weight of the polymeric matrix, in particular greater than 80 % by weight, and even more particularly greater than 90 % by weight.

9. The pharmaceutical composition according to any one of claims 4 to 8, wherein the drug loading content or percentage weight ratio between the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof and the total weight of the microparticles ranges from 2% to 45%, in particular from 5% to 40%, more particularly from 10% to 35%.

10. Pharmaceutical composition under the form of a powder comprising controlled release microparticles comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, and a controlled release polymeric matrix, wherein
- said controlled release microparticles have a mean particle size greater than or equal to 2 µm, in particular ranging from 2 µm to 150 µm, more particularly ranging from 10 µm to 100 µm, even more particularly ranging from 10 µm to 80 µm,
- said controlled release polymeric matrix comprises at least a poly(lactic-co-glycolic acid) copolymer, and
- the drug loading content or percentage weight ratio between the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof and the total weight of the microparticles ranges from 2% to 45%, in particular from 5% to 40%, more particularly from 10% to 35%.

11. The pharmaceutical composition according to any one of claims 1 to 10, wherein it is under the form of a sterile and injectable or implantable dosage form selected from a solution, a suspension, a powder, a solid implant, a semi-solid implant, a microporous implant and an in-situ forming depot.

12. The pharmaceutical composition according to claim 11, in particular under the form of a sterile and injectable suspension, wherein it is obtained by mixing a controlled release dosage form, an immediate release dosage form or a mixture of a controlled release dosage form and an immediate release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a pharmaceutically acceptable salt thereof, in particular microparticles comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, more particularly as defined in any one of claims 3 to 9 with an aqueous injection vehicle, and wherein the pharmaceutical composition optionally comprises an excipient selected from a group consisting of a tonicity-enhancing agent, a wetting-agent, a viscosity-enhancing agent, a density-enhancing agent or a mixture thereof.

13. The pharmaceutical composition according to claim 11 or claim 12, wherein the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof is present in an amount ranging from 2 mg to 63 mg equivalent free acid per ml of the sterile and injectable dosage form, in particular a suspension, in particular from 8 mg to 55 mg equivalent free acid per ml, more particularly from 12 mg to 47 mg equivalent free acid per ml.

14. A kit or article of manufacture comprising, in separate compartments (i) an aqueous injection vehicle and (ii) a controlled release dosage form, an immediate release dosage form or a mixture of a controlled release dosage form and an immediate release dosage form comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof, in particular as claimed in any one of claims 3 to 9 or powder as defined in claim 10, wherein the pharmaceutical composition optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intramedullary injection.

15. The pharmaceutical composition as defined in anyone of claims 1 to 13 or as obtained by mixing the two compartments of the kit as defined in claim 14, for use once or several times by intramedullary injection or intramedullary implantation in a patient prior to or during or after an autologous or allogeneic hematopoietic stem cell transplantation (HSCT), in particular a human patient, for improving the homing, engraftment and long - term expansion and proliferation of hematopoietic stem cells.

16. The pharmaceutical composition for use as defined in claim 15, using an implant in a patient prior to or during or shortly after an autologous or allogeneic hematopoietic stem cell transplantation (HSCT), in particular a human patient, for improving the homing, engraftment and long - term expansion and proliferation of hematopoietic stem cells.

17. The pharmaceutical composition for use according to claim 15 or claim 16, wherein for the hematopoietic stem cell transplantation, the hematopoietic stem cells are selected from bone marrow cells, peripheral blood stem cells, cord blood cells, cultured stem cells, including cultured stem cells after gene therapy.

18. The pharmaceutical composition for use according to any one of claims 15 to 17, wherein said composition is injected or implanted in the bone marrow within about 96 hours prior to and 96 hours after transplantation, in particular within 96 hours prior to and 24 hours after transplantation, more particularly within 96 hours prior to transplantation.

19. The pharmaceutical composition for use according to any one of claims 15 to 18 for decreasing graft failure, for decreasing the occurrence of Poor Graft function (PGF), for decreasing the occurrence of Graft-versus-host disease (GvHD), for enhancing overall survival and donor chimerism, and/or for promoting hematopoietic recovery and hematopoietic stem cell transplantation success rate.

20. The pharmaceutical composition for use as defined in anyone of claims 15 to 19, in the prevention and/or treatment of non-malignant blood disorders such as severe aplastic anemia, and hemoglobinopathies, in particular Sickle cell disease and beta-Thalassemia and/or in the prevention and/or treatment of malignant hemopathies such as myeloma, lymphoma, leukemia, such as acute myeloid leukemia, acute lymphoblastic leukemia, chronic lymphocytic leukemia, and chronic myeloid leukemia, Waldenstrom macroglobulinemia, myeloproliferative neoplasms, and myelodysplastic syndrome, in particular leukemia and/or in the prevention and/or treatment of primary immunodeficiencies, autoimmune diseases or inborn errors of metabolism.

21. The pharmaceutical composition for use according to any one of claims 15 to 20, wherein the time required to release 80% by weight of the 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid or a derivative or a conjugate or a pharmaceutically acceptable salt thereof is greater than 4 day and may reach 30 days preferably 15 days, wherein the 3'-[(2Z)-[1-(3,4-dimethylphenyl)-1,5-dihydro-3-methyl-5-oxo-4H-pyrazol-4-ylidene]hydrazino]-2'-hydroxy-[1,1 '-biphenyl]-3-carboxylic acid or a pharmaceutically acceptable salt thereof is present at a dose ranging from 10 mg to 570 mg equivalent free acid form.

22. The pharmaceutical composition for use according to anyone of claims 15 to 21, wherein the patient is treated prior to or after the intramedullary administration of the pharmaceutical composition as defined in any one of claims 1 to 13 or as obtained by mixing the two compartments of the kit as defined in claim 14, with at least one therapy selected from radiation therapy, radiomimetic chemotherapy and mixtures thereof.
